# EUROPEAN PATENT SPECIFICATION

(11) **EP 3 000 874 B1**
(45) Date of publication and mention of the grant of the patent: **18.09.2024**
(21) Application number: 15186997.1
(22) Date of filing: 25.09.2015
(51) Int. Cl.: A61P 15/08, C12N 5/071

(54) **SEX SORTED SPERM DEMONSTRATING A DOSE RESPONSE AND METHODS OF PRODUCING SEX SORTED SPERM DEMONSTRATING A DOSE RESPONSE**
GESCHLECHTSSORTIERTES SPERMA, DAS EINE DOSISREAKTION DEMONSTRIERT UND VERFAHREN ZUR HERSTELLUNG VON GESCHLECHTSSORTIERTEM SPERMA, DAS EINE DOSISREAKTION DEMONSTRIERT
SPERME TRIÉ SELON LE SEXE PRÉSENTANT UNE RÉPONSE À UNE DOSE ET PROCÉDÉS DE PRODUCTION DE SPERME TRIÉ SELON LE SEXE PRÉSENTANT UNE RÉPONSE À UNE DOSE

(30) Priority: 26.09.2014 US 201462056364 P
(43) Date of publication of application: 30.03.2016
(73) Proprietor: Inguran, LLC, Navasota, TX 77868 (US)
(72) Inventor: VISHWANATH, Ramakrishnan, 3283 Hamilton (NZ); EVANS, Kenneth Michael, Navasota, TX 77868 (US); GILLIGAN, Thomas B., Navasota, TX 77868 (US); MORENO, Juan, Navasota, TX 77868 (US); LENZ, Richard, Eastman, WI 54626 (US); GONZALEZ-MARIN, Clara, Navasota, TX 77868 (US)
(74) Representative: Jacob, Reuben Ellis

(56) References cited:
- US-A1- 2014 099 627
- US-A1- 2014 099 628
- A. GAVIRAGHI ET AL: "Minimum number of spermatozoa per dose in Mediterranean Italian buffalo (Bubalus bubalis) using sexed frozen semen and conventional artificial insemination", THERIOGENOLOGY, vol. 79, no. 8, 1 May 2013 (2013-05-01), US, pages 1171 - 1176, XP055241182, ISSN: 0093-691X, DOI: 10.1016/j.theriogenology.2013.02.014
- KLINC P ET AL: "Insemination with sex sorted fresh bovine spermatozoa processed in the presence of antioxidative substances", REPRODUCTION IN DOMESTIC ANIMALS, vol. 42, no. 1, February 2007 (2007-02-01), pages 58 - 62, XP002752961, ISSN: 0936-6768
- SEIDEL G E ET AL: "Current status of sexing mammalian spermatozoa", REPRODUCTION, BIOSCIENTIFICA LTD, GB, vol. 124, no. 6, 1 December 2002 (2002-12-01), pages 733 - 743, XP002626262, ISSN: 1470-1626
- M F SÁ FILHO ET AL: "Sex-sorted sperm for artificial insemination and embryo transfer programs in cattle", ANIM. REPROD, 15 July 2014 (2014-07-15), pages 217 - 224, XP055240970, Retrieved from the Internet <URL:www.cbra.org.br/pages/publicacoes/animalreproduction/issues/download/v11n3/pag217-224%20%28AR689%29.pdf> [retrieved on 20160113]
- R VISH ET AL: "SEXED SPERM VS CONVENTIONAL SPERM - A COMPARATIVE DISCUSSION", PROCEEDINGS, 1 January 2015 (2015-01-01), pages 250, XP055241256, Retrieved from the Internet <URL:appliedreprostrategies.com/2015/documents/proceedings/16bVishwanath-pg250-256.pdf> [retrieved on 20150115]

## Description

### TECHNICAL FIELD

Generally, the inventive technology relates to staining and sorting methods, such as those employed for sorting sperm, and more particularly relates to sperm sorting methods and flow cytometer methods that improve the efficiency and recovery associated with sex sorting sperm, as well as, the improved dose response in sex-sorted sperm.

### BACKGROUND

The most widely used sperm sorting methods rely on the detection of quantifiable differences in the DNA content of X-chromosome bearing sperm and Y-chromosome bearing sperm. Various modifications to flow cytometers for this purpose are described in U.S. Patents 5,135,759, 6,263,745, 7,371,517 and 7,758,811. In many species, the difference in DNA content can be small. In bovine, for example, Holstein bulls have about a 3.8% difference in DNA content, while Jersey bulls have about a 4.1% difference. The inexact nature of stoichiometric DNA staining makes these minor variations difficult to ascertain and requires vigorous staining protocols.

While the fluorescent dye Hoechst 33342 is suitable for distinguishing such variations in non-toxic concentrations, sperm must be incubated at elevated temperatures and at an elevated pH for Hoechst 33342 penetration to provide uniform staining. Sperm are relatively fragile cells that lack the capacity to replicate and generally demonstrate a short life span. As such, injuries imposed by each of elevating sperm temperature and changing the sperm pH may result in a significant detriment to sperm health. Additionally, the pressure and sheering forces applied to sperm within a flow cytometer may further compromise sperm membranes. The sorting process further includes exposing sperm cells to a UV laser at an interrogation stage, and to an electrical charge in order to deflect droplets containing sperm cells to be collected. Finally, once sorted sperm is ejected from a flow cytometer they impact a collection media at velocities around 80-90km/h. The injuries imposed at each of these steps in the flow sorting process negatively impacts sperm health and may accelerate the deterioration of sperm membranes further reducing the already limited shelf life of viable sperm for use in artificial insemination or other assisted reproductive procedures.

A major drawback of utilizing sex-sorted sperm in the assisted reproductive technologies is the reduced fertility associated with sex-sorted sperm. Studies suggest the fertility of sperm sex-sorted by flow cytometry is about 75-80% of conventional un-sorted semen. Further, increasing the amount of sex sorted sperm utilized in AI does not fully compensate for the subfertility. DeJarmette et al., "Effects of sex-sorting and sperm dosage on conception rates of Holestien Heifers: Is comparable fertility of sex-sorted and conventional semen plausible?" Journal of Dairy Science. Vol. 94, pgs. 3477-3483, (2011). In addition, the following publications relate to methods for sorting sperm cells: A. Gaviraghi et al, Theriogenology, Volume 79 (No 8), 01 May 2013, pages 1171 to 1176; Klinc P et al, Reproduction in Domestic Animals, Volume 42 (No 1), February 2007, pages 58 to 62; US 2014/099627; Seidel G E et al, Reproduction Bioscientifica Ltd, GB, Vol 124 (No 6), 01 December 2002, pages 733 to 743; and US 2014/099628.

### SUMMARY OF THE INVENTION

According to a first aspect of the invention there is provided method of producing sex sorted bovine sperm suitable for insemination having an improved dose response comprising a) extending a bovine sperm sample with a buffering holding media at a sperm sample to buffering holding media ratio between about 1:1 and 1:10, b) adjusting the concentration of the extended sperm sample to a target concentration range and adjusting the pH of the sperm sample towards a predetermined pH by centrifuging the extended sperm sample and removing supernatant to a concentration between about 2400 million sperm per ml and about 500 million sperm per ml, the predetermined pH being a target pH range from 6.8 to 7.4, c) diluting the reconcentrated sperm with a staining media to a concentration between 80 million sperm per ml and 320 million sperm per ml, d)staining the reconcentrated sperm sample with a DNA selective dye, e) maintaining the pH of the sperm sample through steps a) to d) at the target pH range, f) sex sorting the stained sperm sample with a flow cytometer into a catch fluid, and g) supplementing each of the buffering holding media, the DNA selective dye and the catch fluid with at least one antioxidant at a concentration between 0.01 mg per ml and 0.5 mg per ml, h) producing an insemination dosage having between 3 million and 4 million sperm and having at least 90% of the fertility of a conventional dose of 15 million sperm.

### BRIEF DESCRIPTION OF THE DRAWINGS

FIG. 1 illustrates a schematic of a flow cytometer for sorting sperm in accordance with certain embodiments described herein.
FIG. 2 illustrates a graphical representation of sort parameters acquired in a flow cytometer while sorting sperm according to various embodiments described herein.
FIG. 3 illustrates a graphical representation of sort parameters acquired in a flow cytometer while sorting sperm according to various embodiments described herein.
FIG. 4 illustrates a graphical representation of sort parameters acquired in a flow cytometer while sorting sperm according to various embodiments described herein.

### MODES FOR CARRYING OUT THE INVENTION

The term "sperm sample" may be understood to broadly refer to sperm cells in any medium including natural fluids, such as seminal plasma. For example, the term sperm sample may encompass raw ejaculate, neat ejaculate, or semen, as well as processed or partially processed sperm suspended in any combination of buffers, extenders, stains, other media, or the like.

As used herein, the term "instrument parameter" should be understood to include settings relating to the analyzing and/or sorting conditions in, of, and relating to an instrument, where such settings may be modified by manual or automatic adjustments to the instrument. In the case of a flow cytometer, or other similar instruments, the instrument parameters may include, sample pressure, sample flow rate, sheath fluid pressure, sheath flow rate, drop drive frequency, drop drive amplitude, coincidence abort logic, gating regions, sorting logic, and other similar settings.

The term "sorting parameters" may include those conditions relating to sorting preformed in a particle sorting instrument. Sorting parameters may include measured sorting parameters in addition to parameters which are determined offline, estimated by an operator, and conditions relating to a sorted population of particles or cells.

"Measured sorting parameters" may include those conditions relating to sorting measured directly, calculated, or determined in a particle sorting instrument while analyzing and/or sorting a population of particles or cells. In the case of a flow cytometer, or other similar instruments, the measured sorting parameters may include: event rate; sort rate; sorting efficiency; abort rate; dead gate percentage; live oriented gate percentage; valley to peak ratio; or the percentage of events in other sorting gates, such as an X-sort gate or a Y-sort gate.

As used herein the term "coincidence event" may be understood as a single event in a particle sorting instrument where one or more particles or cells are too close to be separated for individual collection, and where only one of the two cells or particles is desirable for collection. In the case of a droplet sorting jet-in-air flow cytometer, a coincident event may occur when two sperm are close enough such that they will end up in the same droplet but only one of those two cells is desired for collection.

The term "sorting efficiency" may be understood to refer to the recovery of particles or cells in terms of the percentage of particles or cells sorted or collected out of a group of cells or particles which are analyzed. The analyzed group of cells may be the total number of cells analyzed or may be a subset of the total number of cells analyzed, such as the analyzed cells determined to be viable or otherwise desirable for analysis and potential collection.

With respect to sorting, the term "productivity," as used herein may be understood to refer to the number of sorted or collected particles or cells per unit time.

With respect to sorting, the term "purity" may refer to an actual or estimated percentage of cells or particles in the population of collected or sorted particles or cells having the characteristic for which the particles were sorted. In the case of sperm, purity may refer to the percentage of X-chromosome bearing sperm in a population sorted for X-chromosome bearing sperm or the percentage of Y-chromosome bearing sperm in a population sorted for Y-chromosome bearing sperm regardless of the viability of the sorted sperm.

The term "antioxidant" refers to any one of a large variety of molecules that either inhibit the oxidation of another molecule, becomes oxidized itself in place of the target substrate, or binds harmful free radical intermediates and interrupts oxidative chain reactions within a cell. Most antioxidants have dual roles; some are enzymes, others are non-enzymatic; some others are vitamins and others are cofactors. Such diversity lauds the diversity of antioxidants, but because of their known ability to minimize cell damage, they are frequently lumped together as a single class of compounds having only a single function, to bind free radicals.

Certain aspects disclosed herein relate to a method of sorting a sperm sample in a particle sorting instrument, however, the methods described are not limited to any specific instruments. Particle sorting instruments may include jet-in-air flow cytometers, such as the Legacy MoFlo^{®} SX flow cytometer, MoFlo^{®} XDP flow cytometer (Beckman Coulter, Miami FL, USA); however, other commercially available flow cytometers could be modified for sperm sorting as well. The jet-in-air flow cytometers may be outfitted with orienting features such as, orienting nozzles for orienting sperm, optics for uniformly illuminating cells, and/or radially uniform optics for collecting fluorescence emissions from all cells regardless of their orientation. Cytometers having different flow chambers may also be used, such as flow cytometers with closed chambers, or cuvettes. Additionally, devices such as microfluidic chips with sorting functions, such as acoustic or fluidic means for deflecting particles into diverging outlets, may be used.

### Pre-Sort Sperm Processing

Certain aspects described herein relate to methods for improving the fertility of sex-sorted bovine sperm, such as methods which result in sex sorted bovine sperm demonstrating a dose response, or which are capable of being sorted at elevated pressures. Each of standardizing sperm prior to staining, staining sperm in a single dilution, and changing the position of a catch tube may independently reduce, or perhaps even eliminate such damage. Some combination of these modifications to existing sorting processes may provide a synergistic benefit.

A bovine sperm sample can be obtained, or provided, by collection of a new ejaculate, by thawing of a frozen sperm sample, or in the act of receiving either. The sperm sample may be in the form of neat semen, extended sperm, frozen-thawed sperm or in combinations thereof. The population of sperm can be obtained at the same location the remaining steps are performed, or can be extended in an appropriate sperm extender for transport to a sorting facility. Once obtained, the sperm can be maintained at room temperature, chilled, or even frozen in an appropriate extender for later use. Sperm for staining and sorting may be acquiring from a mammal, or may be acquired sperm from storage, such as a frozen or chilled straw obtained from storage. Alternatively, frozen or extended sperm may be pooled.

The population of sperm can originate from mammals, such as mammals listed by c.

At the time of collection, or thawing, or even pooling, sperm may be checked for concentration, pH, motility, and/or morphology. Additionally, antibiotics may be added prior to further processing steps.

In one embodiment, once obtained, sperm may optionally be standardized in a target concentration range and/or towards a target pH range. As used herein, "standardizing" may be understood as bringing characteristics of an ejaculate into a target range or near to said target range. While bovine ejaculates, for example, may vary a great deal in pH and sperm concentration, the step of standardizing sperm concentration or pH, may include the addition of buffering holding media that serves to both standardize the pH and buffer against the tendency of ejaculates to become more acidic over time. As a non-limiting example, the pH and concentration of a sperm sample may be standardized pre-sort with a buffering holding solution having a target pH. The sperm sample may be diluted in the buffering holding media at ratios between 1:1 and 1:10, or perhaps at 1:3. The buffered sperm sample may then be centrifuged and supernatant may be removed to reach a target concentration range. Alternatively, the buffered sperm sample may be centrifuged to a pellet and resuspended in buffering holding media, or another similar extender.

Each of the predetermined concentration and pH may be specific to different species, or even to different breeds of animals within a species. In one embodiment, a sperm sample may be combined with an initial extender, such as a buffering holding media in the form of a high capacity buffer or an extender having a large pH buffering capacity. The buffering holding media, and other medias described later, may include TRIS citrate, sodium citrate, sodium bicarbonate, HEPES, TRIS, TEST, MOPS, KMT, TALP, and combinations thereof. Other extenders having a buffer with a high capacity for buffering pH may also be employed, and may be used in combination with additional components which promote sperm viability. As an example of an additive, protein may be incorporated in the form of egg yolk, milk, lipoproteins, lecithin, casein or albumin or other protein sources. An energy source may also be incorporated in the form of a monosaccharide such as fructose, glucose, or mannose, or even a disaccharide or trisaccharide. Additionally, antioxidants and antibiotics may be employed in the buffering holding media to promote sperm viability. Additional additives, such as Bovine Serum Albumin (BSA), may also be supplemented in the initial extender, or buffering holding media.

The buffering holding media may be set at a predetermined target pH to standardize the pH of all obtained sperm samples, such as a pH between about 6.8 and 7.4. For example, the buffering holding media may be adjusted to a pH of 7.2. Additionally, semen may become increasingly acidic over time, possibly due to proteins in the seminal fluid, or due to acidic products of metabolism or byproducts of dead or dying cells. The buffering holding media introduces enough free proton (i.e H⁺) binding sites to maintain pH near a target pH. Even in light of the natural tendency for sperm to become more acidic over time, the buffering holding media provides uniform starting point for stabilizing the pH of multiple sperm sample throughout additional processing steps.

The buffering holding media may include antibiotics to prevent the proliferation of bacteria. As non-limiting examples, tylosin, gentamicin, lincomycin, linco-spectin, spectinomycin, penicillin, streptomycin, and combinations thereof, may be incorporated into the buffering holding media.

Antioxidants may also be incorporated into the buffering holding media for reducing free radicals and oxidative stresses. While the instant discussion relates to the use of antioxidants in a buffering holding media, antioxidants may be incorporated into multiple stages of the sperm sorting process, independently or in combination, as described in International Patent Application WO2012167151. According to the present invention, each of the buffer holding media, the DNA selective dye and the catch fluid are supplemented with at least one antioxidant. A non-limiting list of antioxidants which may be incorporated includes: catalase, SOD, an SOD mimic, glutathione, glutathione reductase, glutathione peroxidase, pyruvate, caproic acid, mercaptoethanol, BHT, lipoic acid, flavins, quinines, vitamin K (and related vitamers), vitamin B12, vitamin B12 vitamers, vitamin E (and related vitamers), tocopherols, tocotrienols, α-tocopheryl, alpha ketoglutarate (AKG), malondialdehyde (MDA), asymmetric dimethylarginine (ADMA) and biologically active derivatives thereof, and combinations thereof.

The concentration of antioxidants may be in the range of 0.01 mg/ml to 0.5 mg/ml, and as non-limiting examples antioxidants listed above may be provided in the concentration 0.01 mg/ml to 5.0 mg/ml; 0.01 mg/ml to 0.25 mg/ml; 0.01 mg/ml to 0.5 mg/ml; 0.01 mg/ml to 1 mg/ml; 0.01 mg/ml to 2.5 mg/ml; 0.01 mg/ml to 5 mg/ml; 0.05 mg/ml to 0.1 mg/ml; 0.05 mg/ml to 1.0 mg/ml; 0.05 mg/ml to 2.5 mg/ml; 0.1 mg/ml to 0.25 mg/ml; 0.1 mg/ml to 0.5 mg/ml; 0.1 mg/ml to 1 mg/ml; 0.1 mg/ml to 2.5 mg/ml; 0.1 mg/ml to 5 mg/ml; 0.15 mg/ml to 0.45 mg/ml; 0.15 mg/ml to 0.5 mg/ml; 0.25 mg/ml to 0.35 mg/ml; 0.25 mg/ml to 0.5 mg/ml; 0.25 mg/ml to 1 mg/ml; 0.25 mg/ml to 2.5 mg/ml; 0.25 mg/ml to 5 mg/ml; 0.35 mg/ml to 0.5 mg/ml; 0.35 mg/ml to 1 mg/ml; 0.35 mg/ml to 2.5 mg/ml; 0.35 mg/ml to 5 mg/ml; 0.5 mg/ml to 1 mg/ml; 0.5 mg/ml to 2.5 mg/ml; 0.5 mg/ml to 5 mg/ml; 1 mg/ml to 2.5 mg/ml; and 1 mg/ml to 5 mg/ml.

The sperm sample may be diluted in the buffering holding media in ratios from about 1:1 to about 1:10. As one example, neat ejaculate may be diluted in a buffering holding media in sperm to buffer ratios between 1:2 and 1:5. For example, the sperm sample may be diluted in the buffering holding medium at a ratio 1:3. The dilution may reduce sperm exposure to some detrimental factors present in the seminal plasma. The diluted sperm sample may then be reconcentrated by centrifugation, removal of supernatant and resuspension. The centrifuged sperm sample may be resuspended in the buffering holding media, or in another similar buffering media in a volume which brings the sperm concentration to or near a target concentration. The target concentration may be selected based on additional sperm processing steps. For example, in the case of sex sorting bovine, sperm may be reconcentrated to between about 2400 million sperm per ml and about 500 million sperm per ml to simulate a natural range of concentrations. Other concentrations, such as between about 1400 million sperm per ml and about 2100 million sperm per ml, or between about 1700 million sperm per ml and about 2100 million sperm per ml may also be utilized for further processing. In this manner seminal plasma originating with the sperm maybe diluted and replaced with one or more buffers, such as a buffering holding media.

Adjusting the sperm concentration and pH may provide a uniform starting point for further processing. For example, a relatively consistent pH and concentration may provide greater predictability in staining sperm, such as with a DNA selective dye. If each sample is adjusted to the same target pH and concentration, fewer trials may be required on each new collection to ensure adequate staining for sex sorting.

A population of sperm will include both X-chromosome bearing sperm and Y-chromosome bearing sperm. Additionally, each of the X-chromosome bearing sperm and the Y-chromosome bearing sperm will include viable sperm and nonviable sperm. Viable sperm can be considered sperm with intact membranes while nonviable sperm can be considered sperm with compromised membranes. The distinction between viable sperm and non-viable sperm in conventional sperm sorting is determined with the inclusion of a quenching dye that permeates membrane compromised sperm. Sperm which tends to be dead or dying absorbs the quenching dye and produces fluorescence signals distinct from the remaining sperm population, whereas sperm having intact membranes tend to be viable and will prevent uptake of the quenching dye. Viable sperm, in the appropriate dosage, will generally be capable of achieving fertilization in an artificial insemination, while nonviable sperm, or membrane compromised sperm, may be incapable of achieving fertilization in an artificial insemination or will have a greatly reduced capacity to do so. However, some sperm capable of fertilization may have compromised membranes, and some sperm with intact membranes may be incapable of fertilization.

Whether extended in a buffering holding media or not, a sperm sample may be stained with a staining solution including a DNA selective dye. In the staining step, at least a portion of the population of sperm is incubated with a staining solution and a DNA selective fluorescent dye in order to stoichiometrically stain the DNA content of each cell in the sperm sample. Hoechst 33342 tends to be less toxic than other DNA selective dyes. The vehicle for delivering this dye may be in the form of a modified TALP buffer adjusted to a pH of about 7.4. Hoechst 33342 is described in US Patent 5,135,759 and is commonly used for this purpose. However, other UV excitable dyes, as well as visible light excitable dyes, fluorescent polyamides, fluorescent nucleotide sequences, and sex specific antibodies could also be used.

Sperm in a natural state is often not readily permeable to such dyes. In order to produce a uniform staining, the first step of staining can include incubating at least a portion of the sperm population at an elevated temperature in a staining solution (sometimes referred to herein as a staining buffer) at an elevated pH in addition to the dye. Examples of appropriate staining solutions can be a TALP, TES-TRIS, TRIS citrate, sodium citrate, or a HEPES based medium, each described in WO2005/095960. An exemplary modified TALP described in WO2001/37655 is illustrated in Table 1.

**TABLE 1 - Modified TALP buffer**

| Ingredient | Concentration |
|---|---|
| NaCl | 95.0 mM |
| KCl | 3.0 mM |
| NaHPO₄ | 0.3 mM |
| NaHCO₃ | 10.0 mM |
| MgCL₂6H₂O | 0.4mM |
| Na Pyruvate | 2.0mM |
| Glucose | 5.0 mM |
| Na Lactate | 25.0 mM |
| HEPES | 40.0mM |
| bovine serum albumin | 3.0 mg/ml |

As one example, the population of sperm, or a portion of the population of sperm, could be diluted with the staining solution to between 640×10⁶ and 40×10⁶ sperm/ml, to between about 320×10⁶ and 80×10⁶ sperm/ml, or to about 160 ×10⁶ sperm/ml in the buffer. The DNA selective fluorescent dye can be added to the sperm suspended in the buffer in concentrations between about 10 µM and 200µM; between about 20 µM and 100µM, or between about 30 µM and 70µM. The pH of the buffer can be between about 6.8 and 7.9; about 7.1 and 7.6; or at about 7.4 in order to help ensure a uniform staining of nuclear DNA. Those of skill in the art will appreciate the pH can be elevated with the addition of NaOH and dropped with the addition of HCl. Optionally, the previously described antioxidants and concentrations may be incorporated into the staining solution.

The sperm sample can be incubated between 30-39°C, between about 32-37°C, or at about 34-36°C. The period of incubation can range between about 20 minutes and about three hours, between about 30 minutes and about 90 minutes, or for about 45 minutes to about 60 minutes. As one example, the population of sperm can be incubated for about 45 minutes at 34°C. Even within a single species, sperm concentration and pH and other factors affecting stainability can vary from animal to animal. Minor variations for incubating sperm between species and even between breeds or animals of the same breed to achieve uniform staining without over staining a population of sperm.

In addition to the DNA selective dye, a quenching dye may be applied for the purpose of permeating membrane compromised sperm and quenching the signals they produce. A quenching dye can be understood to include dyes which differentially associate with membrane compromised sperm. It may be that these dyes enter membrane compromised sperm more easily because the membranes are breaking down or otherwise increasingly porous. It may also be that quenching dyes readily enter all sperm membranes and that healthy sperm actively pump quenching dyes out faster than membrane compromised sperm. In either case, the sperm with which the quenching dyes associate includes a large portion of dead and dying sperm, although not necessarily all dead and dying sperm. The quenched signals produced from membrane compromised sperm having an association with quenching dye are distinct enough from the signals of healthy sperm that they may be removed from the further analysis and sorting applied to viable sperm.

In one embodiment, the quenching dye and the DNA selective dye are applied together in a single dilution. In this embodiment, the quenching dye is incubated along with the DNA selective dye at an elevated temperature in the staining solution. As an example, the staining solution may be a modified TALP with a pH of 7.4. However, other stains may be employed including a TES-TRIS, TRIS citrate, sodium citrate or a HEPES based medium having the DNA selective dye and the quenching dye and pH may range between about 7.0 and 7.8. A synergy may exist when sperm is standardized at an elevated pH of about 7.2 before staining at a pH of 7.4. In this way, the pH to which the sperm is exposed remains in a constant range with minimal variations. Because both the staining solution and the buffering holding media have high buffering capacities, it is believed the natural tendency of sperm to become more acidic over time can be avoided. Additionally, by minimizing the changes in pH endured by the sperm, it is believed the sperm are in a healthier condition to better face various pressures and stresses endured by sperm in the sex sorting process, including, but not limited to additional stresses and shearing forces induced in flow cytometers operated over 40 psi. Staining sperm in a single dilution may help sperm better survive sorting at elevated sheath fluid pressures, such as sheath fluid pressures greater than 40 psi, sheath fluid pressures between 40 and 65 psi, between 50 and 60 psi, or at about 60 psi.

The quenching dye may be added in a second staining step that further reduces the concentration of sperm in the sperm sample. The pH of the second staining solution may be targeted to achieve a target pH in the final sperm sample. Non-limiting examples of two step staining processes are described in published PCT International Application WO 2011/123166 and International Application PCT/US12/58008.

The staining solution may be supplemented additives, such as an antioxidant in the previously described concentration ranges. Elevated temperatures may increase free radicals and oxidative stresses endured by sperm being stained. Accordingly, antioxidants may serve to neutralize free radicals and reduce the oxidative stresses endured by the sperm being stained. A non-limiting list of antioxidants which may be incorporated in the staining process includes: catalase, SOD, an SOD mimic, glutathione, glutathione reductase, glutathione peroxidase, pyruvate, caproic acid, mercaptoethanol, BHT, lipoic acid, flavins, quinines, vitamin K (and related vitamers), vitamin B 12, vitamin B12 vitamers, vitamin E (and related vitamers), tocopherols, tocotrienols, α-tocopheryl, alpha ketoglutarate (AKG), malondialdehyde (MDA), asymmetric dimethylarginine (ADMA) and biologically active derivatives thereof, and combinations thereof. Any of the previously described concentrations may be used.

Certain aspects of the pre-sorting methodologies described above may be combined in synergistic manners. For example, sperm may be standardized to target pHs and concentrations, maintained within a relatively narrow range of pHs throughout the pre-sorting process and exposed to uniform levels of antioxidants throughout the pre-sorting process. Alone or in combination, these modifications to the sorting process may help sperm better survive flow cytometers sorting. Sperm may then be sorted at higher concentrations and at higher pressures, perhaps even pressure previously considered detrimental to sperm health, to achieve improved efficiencies and throughput. Finally, the improved sperm health achieved in the examples below may also provide sex-sorted sperm which demonstrates a dose response in artificial insemination. Sex-sorted sperm capable of demonstrating a dose response may then be utilized in artificial insemination at dosages with comparable fertility to conventional unsorted sperm.

### Sperm Sorting

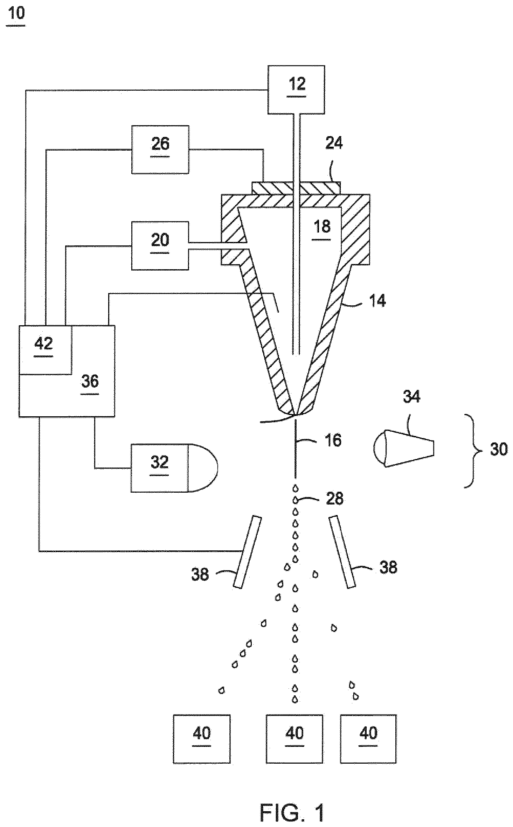

Whether standardized to a predetermined pH and concentration or not, and whether stained in a single dilution or in two dilutions, the sperm sample can be sorted by a particle sorting instrument, such as flow cytometer. Referring to FIG. 1, a jet-in-air flow cytometer (10) is illustrated, although sorting may be performed with microfluidic chips or other types of flow cytometers, including flow cytometer having closed chambers and cytometers and cytometers incorporating ablating lasers. The flow cytometer (10) includes a cell source (12) for producing a flow of sperm sample, such as a flow of stained sperm sample for sorting. The rate at which the sperm sample is delivered to the nozzle (14) may be considered the sample flow rate, and may be determined by a sample pressure. The flow of stained sperm sample is deposited within a nozzle (14) and introduced into, or flowed into, a fluid stream (16) of sheath fluid (18). The sheath fluid (18) can be supplied by a sheath fluid source (20) so that as the cell source (12) supplies the sperm into the sheath fluid (18) they are concurrently fed through the nozzle (14). The sheath fluid (18) may be supplied at a sheath flow rate which is determined by a sheath fluid pressure. In this manner the sheath fluid (18) forms a fluid stream coaxially surrounding the sample having stained sperm which exits the nozzle (14) at the nozzle exit orifice (22). By providing an oscillator (24) which may be precisely controlled with an oscillator control (26), pressure waves may be established within the nozzle (14) and transmitted to the fluids exiting the nozzle (14) at nozzle exit orifice (22). In response to the pressure waves, the fluid stream (16) exiting the nozzle exit orifice (22) eventually forms regular droplets (28) at precise intervals. The frequency, and to some extent the shape of the formed droplets may be controlled by a drop drive frequency and drop drive amplitude supplied to the oscillator (24) or the oscillator controller (26).

Each formed droplet retains sheath fluid and sperm sample that previously formed a portion of the fluid stream (16). Because the stained sperm are surrounded by the fluid stream (16) or sheath fluid environment, the droplets (28) ideally contain individually isolated sperm. However, the sample concentration, sample pressure, the diameter of the nozzle exit orifice (22) and other instrument parameters dictate the frequency with which multiple cells will regularly occupy a single droplet, as well as the percentage of droplets containing sperm.

The flow cytometer (10) acts to sort droplets based on the characteristics of sperm predicted to be contained within the droplets. This can be accomplished through a cell sensing system (30) in communication with an analyzer (36). The cell sensing system (30) includes at least one sensor (32) responsive to the cells contained within fluid stream (16). As one example, two orthogonal PMTs may be incorporated into a sperm sorting flow cytometer for detecting fluorescence at 0 degrees and 90 degrees, although other sensor configurations can readily be employed, such as those described in WO2010/021627.

The cell sensing system (30) provides data to the analyzer (36), which may cause an action depending upon the relative presence or relative absence of a characteristic of cells in the fluid stream (16). Certain characteristics, such as the relative DNA content of sperm, can be detected through excitation with an electromagnetic radiation source (34), such as a laser generating an irradiation beam to which the stained sperm are responsive. The electromagnetic radiation source (34) can be a laser operated at UV wavelength, such as at about 355 nm. An example of such a laser can be a Vanguard 350 (available from Spectra-Physics), which operates at 350mW. Various optics may be employed to shape the beam profile of the laser, split the beam to more than one stream, or reduce the beam power at a stream. Non-limiting examples of such optics can be found in WO/2004/104178 and WO/2001/85913.

The characteristics of individual sperm, particularly the presence of an X-chromosome or a Y-chromosome can be determined from the detected fluorescence produced in response to the electromagnetic radiation source (34). In particular, configurations of the cell sensing system (30) may be in communication with an analyzer (36) for providing a variety of fluorescence in formation, such as the forward fluorescence of an event, the side fluorescence of an event, or the amount of scatter associated with an event. The analyzer (36) may include written instructions for analyzing the signals produced by the one or more sensors (32) in the cell sensing system (30). The DNA selective fluorescent dye binds stoichiometrically to sperm DNA. Because X-chromosome bearing sperm contain more DNA than Y-chromosome bearing sperm, the X-chromosome bearing sperm can bind a greater amount of DNA selective fluorescent dye than Y-chromosome bearing sperm. Thus, by measuring the fluorescence emitted by the bound dye upon excitation, it is possible to differentiate between X-bearing spermatozoa and Y-bearing spermatozoa. Distinctions, such as sperm which is viable or not viable, may be differentiated in addition to oriented and unoriented sperm by the analyzer (36) according to sorting logic incorporated gating regions.

In order to achieve separation and isolation based upon stained sperm characteristics, emitted light can be detected by the sensor (32) and the information fed to an analyzer (36) coupled to a droplet charger which differentially charges each droplet (28) based upon the characteristics of the stained sperm contained within that droplet (28). In this manner the analyzer (36) acts to permit the electrostatic deflection plates (38) to deflect droplets (28) based on whether or not they contain the appropriate particle or cell.

As a result, the flow cytometer (10) acts to separate stained sperm by causing the droplets (28) containing sperm to be directed to one or more collection containers (40). For example, when the analyzer differentiates sperm based upon a sperm characteristic, the droplets entraining X-chromosome bearing spermatozoa can be charged positively and thus deflect in one direction, while the droplets entraining Y-chromosome bearing spermatozoa can be charged negatively and thus deflect the other way, and the wasted stream (that is droplets that do not entrain a particle or cell or entrain undesired or unsortable cells) can be left uncharged and thus collected from an undeflected stream into a suction tube or the like. Alternatively, one of the X-chromosome bearing sperm or the Y-chromosome bearing sperm may be collected, while the other is discarded with waste. Once deflected, droplets may then be collected in collection containers which include a catch fluid. The catch fluid may comprise an extender, similar to the buffering holding media or similar to a subsequent extender which may be used for cooling or freezing sorted sperm. By way of an example, the catch fluid may comprise a buffering component such as TRIS citrate, sodium citrate, sodium bicarbonate, HEPES, TRIS, TEST, MOPS, KMT, TALP, or combinations thereof. Other buffers having a high capacity for buffering pH may also be employed, any of which may be used in conjunction with additional components that promote sperm viability. As an example of an additive, protein may be incorporated in the form of egg yolk, milk, lipoproteins, lecithin, casein or albumin or other protein sources. An energy source may also be incorporated in the form of a monosaccharide such as fructose, glucose, or mannose, or even a disaccharide or trisaccharide. Additionally, antioxidants and antibiotics may be employed in the initial extender to promote sperm viability.

A non-limiting list of antioxidants which may be incorporated in the catch fluid includes: catalase, SOD, an SOD mimic, glutathione, glutathione reductase, glutathione peroxidase, pyruvate, caproic acid, mercaptoethanol, BHT, lipoic acid, flavins, quinines, vitamin K (and related vitamers), vitamin B12, vitamin B12 vitamers, vitamin E (and related vitamers), tocopherols, tocotrienols, α-tocopheryl, alpha ketoglutarate (AKG), malondialdehyde (MDA), asymmetric dimethylarginine (ADMA) and biologically active derivatives thereof, and combinations thereof.

The concentration of antioxidants may be in the range of 0.01 mg/ml to 0.5 mg/ml, and as non-limiting examples antioxidants listed above may be provided in the concentration 0.01 mg/ml to 5.0 mg/ml; 0.01 mg/ml to 0.25 mg/ml; 0.01 mg/ml to 0.5 mg/ml; 0.01 mg/ml to 1 mg/ml; 0.01 mg/ml to 2.5 mg/ml; 0.01 mg/ml to 5 mg/ml; 0.05 mg/ml to 0.1 mg/ml; 0.05 mg/ml to 1.0 mg/ml; 0.05 mg/ml to 2.5 mg/ml; 0.1 mg/ml to 0.25 mg/ml; 0.1 mg/ml to 0.5 mg/ml; 0.1 mg/ml to 1 mg/ml; 0.1 mg/ml to 2.5 mg/ml; 0.1 mg/ml to 5 mg/ml; 0.15 mg/ml to 0.45 mg/ml; 0.15 mg/ml to 0.5 mg/ml; 0.25 mg/ml to 0.35 mg/ml; 0.25 mg/ml to 0.5 mg/ml; 0.25 mg/ml to 1 mg/ml; 0.25 mg/ml to 2.5 mg/ml; 0.25 mg/ml to 5 mg/ml; 0.35 mg/ml to 0.5 mg/ml; 0.35 mg/ml to 1 mg/ml; 0.35 mg/ml to 2.5 mg/ml; 0.35 mg/ml to 5 mg/ml; 0.5 mg/ml to 1 mg/ml; 0.5 mg/ml to 2.5 mg/ml; 0.5 mg/ml to 5 mg/ml; 1 mg/ml to 2.5 mg/ml; and 1 mg/ml to 5 mg/ml.

The antioxidants may be supplemented in each of the buffering holding media, the staining solution and the catch fluid. The antioxidants may comprise the same additives or combination of additives and may be present in each similar concentrations in each of the buffering holding media, the staining solution and the catch fluid. In this way oxidative stress may be mitigated in sperm throughout the sorting process. In this manner aspects of the sorting methodologies described above may be combined in synergistic manners throughout the entire sorting process. For example, sperm may be standardized to target pHs and concentrations and maintained within a relatively narrow range of pHs throughout the sorting process. Sperm may also be exposed to uniform levels of antioxidants throughout the sorting process. Alone or in combination, these modifications may help sperm better survive flow cytometers sorting allowing sperm to be sorted at higher pressures, perhaps even pressure previously considered detrimental to sperm health, or to achieve a dose response with sex-sorted sperm in artificial insemination.

A controller (42) may form a portion of the analyzer (36) or may be a component external to the analyzer (36). The illustrated controller (42) may also represent a collection of individual controllers. The controller (42) may receive signals or instructions from the analyzer (36) and in response may modify one or more instrument parameters, such as the sample flow rate, sample pressure, sheath flow rate, sheath fluid pressure, drop drive frequency, or drop drive amplitude and the like. The controller (42) may also provide an interface for operator input to manually adjust the sample flow rate, sample pressure, sheath flow rate, sheath fluid pressure, drop drive frequency, drop drive amplitude and the like. The analyzer (36) may include written instructions for modifying the instrument parameters in response to measured sorting parameters, or modifications to instrument parameters may be manually performed by an operator adjusting various settings. The modifications to instrument parameters may be carried out in the analyzer (36) such as for changing sorting logic, abort logic, sorting regions, or gate regions and other parameters specific to making sort decisions in the analyzer. Additional modifications to instrument parameters may be effected by a controller (42), for controlling various external components to the analyzer, such as for controlling the sample pressure, sample flow rate, sheath fluid pressure, sheath flow rate, drop drive frequency, and drop drive amplitude.

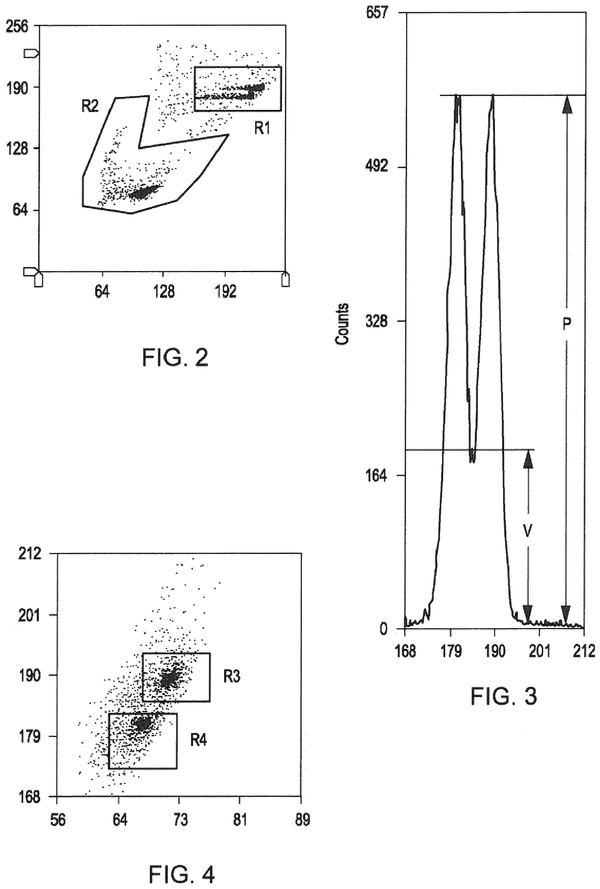

FIG. 2 illustrates a representative bivariate histogram plot of side fluorescence and forward fluorescence from a jet-in-air flow cytometer of stained sperm, which may be generated by an analyzer (36). The visual representation of data may be used by an operator to receive feedback relating to the sample undergoing sorting and to graphically demonstrate certain aspects of the current sorting logic. R1, for example, can be seen as a gating region which may be applied to the sort logic of the flow cytometer. Additional numerical output may be provided in a display of the analyzer (36). Such numerical output may be in the form of measured sorting parameters, such as an event rate, an abort rate, sort rate, sorting efficiency, or the percentage of particles in any region or gate. R1 is illustrated as a region which may be considered the live oriented region, because the boundaries of R1 include two dense populations of cells which reflect a closely related X-chromosome bearing population of sperm and Y-chromosome bearing population of sperm. R2 is a gating region set around the non-viable sperm, or the membrane compromised sperm whose fluorescence is quenched by a quenching dye. While a variety of sort logics may be employed, two strategies relating to R1 and R2 might be a first step in a sorting logic whereby all events falling in R1 are accepted for further processing or gating. Alternatively, all events falling outside of R2 are accepted for further processing or gating.

FIG. 3 illustrates a univariate plot in the form of a histogram that may be produced by the analyzer (36) and generated into a graphical presentation for an operator. The data illustrated in FIG. 3 may represent the number of occurrence of peak signal intensities from the side or forward fluoresce within a certain period. In the case of sperm, X-chromosome bearing sperm and Y-chromosome bearing sperm tend to have peak intensities that vary by between 2 and 5%, depending on the species, and this difference is reflected in the bimodal distribution of peak intensities seen in FIG. 2. Because X-chromosome bearing sperm and Y-chromosome bearing sperm tend to have differing fluorescence values, each of the peaks represents either X-chromosome bearing sperm of Y-chromosome bearing sperm. Based on the sort logic applied within the analyzer (36), the population of cells in the histogram may be only those cells which were determined to be viable oriented cells, such as those falling into R1 in FIG. 2, or they may represent cells which were not determined to be dead or undesirable, such as every event except those falling in R2. A variety of sorting parameters may be derived from the information contained within this histogram. For example, the level of distinctiveness between the two peaks may provide an indication of what a sorted purity may look like. FIG. 3 further illustrates relative intensity measurements at the lowest point between the two groups, which may be considered a value V and a second relative intensity at the peak or peaks of the histogram at P. A visual inspection of a histogram may provide an operator with an idea of how a flow cytometer is performing, but computer executed instructions for determining a P value, a V value, and a ratio of V to P has not been implemented in commercial sperm sorters. The valley to peak ratio, may be determined as a measured sorting parameter periodically during the course of sorting. The valley to peak ratio, while not the necessarily completely determinative of sorting purities, may provide a means for quickly estimating purity values, either automatically by the execution of written instruction in the analyzer (36), or manually by visual inspection of an operator. Alternatively, the inverse relationship, namely a peak to valley ratio, provides similar information as the inverse value.

Turning to FIG. 4, a second bimodal plot may be generated by the analyzer (36) in response to signals acquired by the cell sensing system (30). The bimodal plot may represent a first axis illustrating the peak intensity value of a forward fluorescence signal or the peak intensity of side fluorescence signal. Like FIG. 3, the data illustrated in FIG. 4 may be gated such that only events falling within R1 in FIG. 2 are included. Alternatively, in the case of sperm, all events which do not fall into the dead gate R2 may also be displayed.

R3 may represent an X-sort gate for collecting X-chromosome bearing sperm. The term X-sort gate may be used interchangeably herein with the term X-gate. With reference to FIG. 4, it may demonstrate how changing the dimensions of the gating regions may affect efficiency, purity, and productivity. If the R3 region were to be expanded, it could be seen that every second more sperm would be sorted as X-chromosome bearing sperm resulting in higher sorting efficiency and higher productivity. However, the expansion of the R3 gate or region would begin to include events having an increasing likelihood of being Y-chromosomes bearing sperm. In order to increase the sorted purity of sperm, the R3 region can be made smaller and/or moved away from the Y-chromosome region. As fewer events fall within the X-sort gate, fewer sperm are sorted in the X-chromosome bearing sperm population and those which are have a greater probability of actually being X-chromosome bearing sperm, meaning the collected purity may be increased. However, both the efficiency, in terms of cells collected, and the productivity, in terms of sorts per second, will decrease as fewer events fall within the R3 region and more coincident events are aborted. Additionally, as other instrument parameters are modified, the illustrated graphs of FIG. 2, FIG. 3, and FIG. 4 may change in shape and nature. For example, increasing a sample pressure or a sample flow rate may result in a reduction in the valley to peak ratio, or may otherwise lessen the bimodal distinction between X-chromosome bearing sperm and Y-chromosome bearing sperm.

### Dose Response and Increased Sexed Doses

Conventional semen can be understood as semen collected according to standard industry practices. As one non-limiting example of a standard industry practice for collecting conventional semen, the methodology described as conventional by DeJarnette et al., "Effects of 2.1 and 3.4 × 106 sex sorted sperm dosages on conception rates of Holstein cows and heifers," Journal of Dairy Science, Vol. 93, pgs. 4079-7085 (2010) provided a 62% conception rate with 15 million conventional (un-sexed) sperm. As illustrated in Example 7, certain sorting methods provided herein generate sex sorted sperm with fertility at 59.9%, or even as high as 66.7%. Compared to a control of 15 million conventional sperm (66.5%), 3 million sex sorted sperm (60.0%) and 4 million sex sorted sperm (66.7%) provided a relative fertility at 90% and 100%, respectively. 4 million sex sorted sperm did not produce a statistically significant loss in fertility (P<0.05) as compared to a dose of 15 million conventional semen. It should be appreciated a large number of factors can influence fertility, such as time or year, ambient temperature, heifers v. cows, and accuracy in detecting estrus, AI technician skill, as well as the particular characteristics of specific breeds, or even different herds or specific animals. Accordingly, fertility can be understood to vary for both sex sorted sperm and conventional sperm for a number of reasons. As such, sexed sperm may be understood to have fertility which is 90% of an un-sexed conventional control. The fertility of sex sorted bovine sperm, sorted according to certain methods described herein, may have fertility of at least about 50%, at least about 55%, at least about 60%, at least about 65%, or even at least about 67% or greater.

Sperm sorted according to certain embodiments described herein may have fertility characteristics including fertility at least 90% as high as the fertility of conventional semen or even as high as conventional semen. As demonstrated in Example 7, doses of 3 million sex sorted sperm were able to achieve 90% of the fertility of conventional semen and doses of 4 million sex sorted sperm were able to achieve the same fertility as doses of 15 million conventional (un-sexed) sperm.

As a non-limiting example, a straw may be filled with between about 3 million sperm and about 4 million sperm, between about 4 million sperm and about 5 million sperm, between about 5 million sperm and about 6 million sperm, between about 6 million sperm and about 7 million sperm, between about 7 million sperm and about 8 million sperm, between about 8 million sperm and about 9 million sperm, between about 9 million sperm and about 10 million sperm, between about 10 million sperm and about 11 million sperm, between about 11 million sperm and about 12 million sperm, between about 12 million sperm and about 13 million sperm, between about13 million sperm and about 14 million sperm, between about 14 million sperm and about 15 million sperm, between about 15 million sperm and about 16 million sperm, between about 16 million sperm and about 17 million sperm, between about 17 million sperm and about 18 million sperm, between about 18 million sperm and about 19 million sperm, and between about 19 million sperm and about 20 million sperm.

While the step of reconcentrating sorted sperm may largely replace extenders and stain utilized throughout the sorting process, there residual amounts of those extenders and the various components thereof may remain. As one example, where an antioxidant is added with an initial extender (buffering holding medium) that antioxidant may be present in a residual amount in the final sperm dosage. Additionally, an antioxidant provided in the step of staining may also be present in a residual amount in the final sperm dosage. Understandably, an antioxidant present in the catch fluid may also be present in the final dosage. In this way, a dosages of sperm, such as between 3 million and 20 million sex sorted sperm, may contain a residual amount of a first antioxidant, a residual mount of a second antioxidant and a residual amount of a third antioxidant. The same antioxidant, or combination of antioxidants, may be added in each of the initial extender, the stain, and the catch fluid and may still be considered a first, second, and third residual antioxidant in the final form.

Once sorted, individual dosages of sperm may be prepared for artificial insemination (AI), *in vitro* fertilization (IVF), or intra-cytoplasmic sperm injection (ICSI). Sperm for use in assisted reproductive technology may typically be stored in straws. Previously, owing to the fact that sex sorted sperm did not provide a dose response, sex sorted sperm was frequently loaded into 0.25 ml straws at concentrations of about 10×10⁶ sperm per ml. Due to a small volume occupied in each straw by plugs for sealing sperm therein, about 2.1 million sperm cells ended up in each straw of sorted sperm.

Between 3 million and 20 million sperm may be used in a dosage for AI or IVF. An antioxidant may be added at an initial stage in a buffering holding media. It may be appreciated a residual amount of the antioxidant may be retained with the sperm sample through the subsequent steps of staining, sorting, and in some cases freezing. In this context, the antioxidant presented in the buffering holding media may found in residual amounts in the final straw and may be referred to as a first residual amount of antioxidant. Similarly, a second residual amount of antioxidants present in the staining solution may be retained with the sperm sample through the sorting, and perhaps the freezing steps. For this reason, a straw may include a second residual amount of antioxidants introduced at the staining step. Additionally, a residual amount of antioxidants present in the catch fluid may be retained with the sperm sample and end up in a straw as a third residual amount of antioxidant. Finally, a fourth amount of antioxidant may be presented before or after a reconcentrating a sperm sample for loading into straws with a medium for freezing. For this reason, the fourth amount of antioxidant may be present in a residual amount or may be present in a concentration between about 0.1mg/ml and about 5mg/ml.

### Example 1

*Collection* - Sperm was collected from five different bulls on a routine collection schedule using an artificial vagina. Each bull was collected two or three times in one day. Of the five bulls, two were Jersey bulls and three were Holstein bulls. All ejaculates contained greater than 60% progressive motility and sperm concentration varied from 857 million sperm per mL to 2480 million sperm per mL. Ejaculates collected from the same bull were pooled then divided into nine sperm samples for collection and staining treatments.

*Sperm processing and staining* - Portions of each bull ejaculate were processed and stained by nine different methods, each described as follows.
(A) Control (no standardization, two step staining) - A control was established which did not include the step of standardizing collected ejaculates and in which the sperm was stained in two steps. Prior to staining, the sperm samples were concentrated to between 1700 million sperm per mL and 1800 million sperm per mL by centrifugation or by the addition of a tris-egg yolk extender having a pH of 6.8, depending on the samples starting concentration.

Sperm in the control group was diluted to 160×10⁶ sperm per ml in a modified TALP buffer, as described in Table 1, at a pH of 7.4. Each sperm sample in the control group was then incubated with 16-17µL of Hoechst 33342 per ml (64-68 µM) of sample for 45 minutes at 34°C. After incubation, an equal volume of a second modified TALP was added reducing the concentration to 80×10⁶ sperm per mL. The second modified TALP includes the components described in Table 1 with the addition of 4% egg yolk, 50 µM yellow food dye No. 6 (20 g/L) and the pH was dropped to 5.5 with the addition of HCl.

(B) Extended (no standardization, two step staining) - In the second group, sperm was not standardized, but was extended with an extender having 20% egg yolk. The sperm was then concentrated to between 1700 million sperm per mL and 1800 million sperm per mL in the same manner described with respect to group (A). The sperm was then diluted to 160×10⁶ sperm per ml in a modified TALP buffer, and stained in the same two step manner described in group (A).

(C) One Step I (no standardization, one step staining with 1% egg yolk) - In a third group sperm was collected and the concentration was adjusted in the same manner as the control group (A). Each sperm sample was then diluted to 160×10⁶ sperm per ml in a modified TALP buffer at a pH of 7.4. The modified TALP buffer was substantially identical to the buffer described in Table 1, except that it additionally included 1% egg yolk and yellow food dye No. 6 at a concentration of 25 µM. Each sperm sample in this group was then incubated with 14-15µL of Hoechst 33342 per ml (56-60 µM) for 45 minutes at 34°C. After incubation, sperm remained at a concentration of 160×10⁶ sperm per ml.

(D) Standardized I (standardized with 3% egg yolk extender, two step staining) - In this group sperm was standardized by adjusting both the pH and sperm concentration prior to staining and sorting. After collection sperm was diluted 1:3 in an initial extender having a pH of 7.2 as well as a high capacity for buffering pH. The high capacity buffer was supplemented with 3% egg yolk. All samples were then centrifuged to bring the sperm concentration down to between 1700 million sperm and 1800 million sperm per mL. The standardized sperm was then stained according to the two step method described in (A).

(E) Standardized II (standardized with 10% egg yolk extender, two step staining) - In this group sperm was standardized by adjusting both the pH and sperm concentration prior to staining in the same manner described in group (D), except that the initial extender was 10% egg yolk.

(F) One Step and Standardized I (standardized with 3% egg yolk extender, one step staining with 1% egg yolk) - In this group sperm was standardized by adjusting both the pH and sperm concentration prior to sorting in the same manner described in group (D). The standardized sample was then stained with a one step staining process as described in group (C).

(G) One Step and Standardized II (standardized with 10% egg yolk extender, one step staining with 1% egg yolk) - In this group sperm was standardized by adjusting both the pH and sperm concentration prior to staining in the same manner described in group (E). The standardized sample was then stained with a one step staining process as described in group (C).

(H) One Step and Standardized III (standardized with 3% egg yolk extender, one step staining with no egg yolk) - In this group sperm was standardized by adjusting both the pH and sperm concentration prior to staining in the same manner described in group (D). The standardized sample was then stained with a one step staining process as described in group (C), except that no egg yolk was added to the one step staining TALP.

(I) One Step and Standardized IV (standardized with 10% egg yolk extender, one step staining with no egg yolk) - In this group sperm was standardized by adjusting both the pH and sperm concentration prior to sorting in the same manner described in group (E). The standardized sample was then stained with a one step staining process as described in group (C) except that no egg yolk was added to the one step staining TALP.

*Sorting and data acquisition* - Each of the stained samples was sorted on a Legacy MoFlo^{®} SX flow cytometer (Beckman Coulter, USA) with a Genesis digital upgrade (Cytonome/ST, Boston MA, USA). Those samples which were stained in a two step process were sorted at the concentration of 80×10⁶ sperm per mL, and those samples which were stained by the one step process were sorted at the concentration of 160 ×10⁶ sperm per mL. Data logged by the flow cytometer was recorded, including information relating to the sort rates and gating of sperm subpopulations. For example, the percentage of sperm gated as dead, as well as the percentages of sperm gated as live-oriented and over ranges were recorded and averaged for the five bulls.

*Results* - A comparison of the percentage of sperm which was orientated, unoriented and dead as determined by the sort parameters established in the flow cytometer are summarized in Table 2 below.

**TABLE 2**

| | %Oriented | %Non-oriented | % Dead | Sort Rate | Overrange |
|---|---|---|---|---|---|
| A) Control | 58.29% | 18.02% | 16.89% | 3500 | 4.32% |
| B) Extended | 60.54% | 20.20% | 8.71% | 3400 | 10.36% |
| C) One Step I | 61.04% | 17.96% | 12.31% | 3500 | 5.65% |
| D) Standardized I | 52.78% | 18.14% | 9.71% | 2900 | 24.73% |
| E) Standardized II | 55.20% | 18.70% | 6.04% | 3200 | 23.44% |
| F) One Step + Standardized I | 57.33% | 20.35% | 5.39% | 3200 | 16.17% |
| G) One Step + Standardized II | 59.99% | 18.89% | 5.19% | 3600 | 16.83% |
| H) One Step + Standardized III | 62.67% | 22.02% | 6.97% | 3800 | 6.23% |
| I) One Step + Standardized IV | 63.49% | 23.16% | 5.61% | 4100 | 5.38% |

As compared to the control (A), the groups One Step I (C), Standardized I (D), and Standardized II (E), each exhibited significantly lower dead populations with reductions of 4.58%, 7.18% and 10.85%, respectively. Based on these improvements, the steps of standardizing sperm samples before staining and modifying the staining process to a single step independently improve the ability of sperm to survive the sorting process. Additionally, One Step and Standardized I (F), One Step and Standardized II (G), One Step and Standardized III (H), and One Step and Standardized IV (I), demonstrate a synergy whereby the combined effect of standardizing an ejaculate and staining the ejaculate in a single step is greater than either improvement individually.

Referring to Table 2, it can be seen that Standardize 1 (D), Standardize II (E), One Step and Standardized I (F), and One Step and Standardized II (G), each appeared to provide significant benefits in terms reducing the number of dead sperm, but the percentage of oriented sperm did not improve. This may be related to the column indicated as over range. While more sperm were gated as live for sorting there appears to be an increase in signals scattered above the sorting gate ranges. This signal may represent sperm which is stuck together or may represent sperm which is bound to egg yolk lipids. In either event, the general pattern emerges that greater quantities of egg yolk reduce dead sperm numbers, but may introduce a new issue and a balance may therefore be required.

Additionally, the each trial incorporating one step staining methodology provided a more efficient means for associating the DNA selective dye Hoechst 33342 with the nuclear DNA of sperm cells. Staining quality was maintained across each tested condition, but the tests including only the single staining step utilized 2µL less Hoechst per mL of sample. The ability to stain with less Hoechst may contribute to overall improved sperm health.

### Example 2

*Collection* - Sperm was collected from six different Jersey bulls on a routine collection schedule using an artificial vagina. All ejaculates contained greater than 65% progressive motility and sperm concentration varied from 765 million sperm per mL to 1710 million sperm per mL. Each Sperm sample was divided into two parts in 15mL tubes for two collection and staining treatments. pH measurements were taken at collection, and at each subsequent processing step.

*Sperm processing and staining* - Portions of each bull ejaculate were processed and stained by two methods for comparison.

Control (no standardization, two step staining) - A control was established which did not include the step of standardizing collected ejaculates and in which the sperm was stained in two steps. Prior to staining, the sperm samples were concentrated to between 1700 million sperm per mL and 1800 million sperm per mL by centrifugation or by the addition of a tris-egg yolk extender having a pH of 6.8, depending on the samples starting concentration.

Sperm in the control group was diluted to 160×10⁶ sperm per ml in a modified TALP buffer, as described in Table 1, at a pH of 7.4. Each sperm sample in the control group was then incubated with 16-17µL of Hoechst 33342 per ml (64-68 µM) of sample for 45 minutes at 34°C. After incubation, an equal volume of a second modified TALP was added reducing the concentration to 80×10⁶ sperm per mL. The second modified TALP includes the components described in Table 1 with the addition of 4% egg yolk, 50 µM red food dye No. 40 (20 g/L) and the pH was dropped to 5.5 with the addition of HCl.

One Step and Standardized (standardized with 10% egg yolk, one step staining with one percent egg yolk) - Sperm was standardized by adjusting both the pH and sperm concentration prior to staining. After collection sperm was diluted 1:3 in an initial extender having a pH of 7.2 as well as a high capacity for buffering pH. The high capacity buffer was supplemented with 1% egg yolk. All samples were then centrifuged to bring the sperm concentration down to between 1700 million sperm and 1800 million sperm per ml.

The sperm samples were then diluted to 160×10⁶ sperm per ml in a modified TALP buffer at a pH of 7.4. The modified TALP buffer was substantially identical to the buffer described in Table 1, except that it additionally included 1% egg yolk and yellow food dye No. 6 at a concentration of 25 µM. Each sperm sample in this group was then incubated with 16-17µL of Hoechst 33342 per mL (64-68 µM) for 45 minutes at 34°C. After incubation, sperm remained at a concentration of 160×10⁶ sperm per mL.

*Sorting and data acquisition* - Each sample was sorted on a MoFlo^{®} SX flow cytometer (Beckman Coulter, USA) with a Genesis digital upgrade (Cytonome/ST, Boston MA, USA). The control was sorted at the concentration of 80×10⁶ sperm per mL, while the standardized sperm was sorted at 160 ×10⁶ sperm per ml. Data was logged by the flow cytometer and then averaged for the 6 bulls.

*Results* - TABLE 3 illustrates the recorded pH of both the control (A) and the standardized ejaculate (B). These Values are reflected in TABLE 3 below. While the standardized ejaculate is subject to an initial increase, a subsequent increase is avoided during staining and the following drop off is also avoided. Additionally, TABLE 4 illustrates similar benefits in the reduction of dead sperm that was seen in Example 1. Specifically, the standardized sample which was stained in one step had 5.67% less dead sperm.

**TABLE 3**

| | Initial | Before Centrifugation | After Centrifugation | During Staining | After staining | Before cytometer |
|---|---|---|---|---|---|---|
| Control (A) | 6.34 | 6.34 | 6.25 | 7.22 | 7.07 | 6.59 |
| Standardized (B) | 6.34 | 7.12 | 6.85 | 7.18 | 6.98 | 6.98 |

**TABLE 4**

| | PV | %Oriented | % Dead | Sort Rate | Duplets/Triplets |
|---|---|---|---|---|---|
| Control | 1.86 | 52.99 | 14.63 | 35.83 | 21.73 |
| Standardized - One Step | 1.97 | 57.22 | 8.96 | 37.00 | 24.59 |
| Difference | 0.11 | 4.23 | -5.67 | 1.17 | 2.86 |

### Example 3

*Collection* - Sperm was collected from three different Jersey bulls and three different Holstein bulls on a routine collection schedule for a total of 17 collections. Each ejaculate was divided for two treatments.

*Sperm processing and staining* - Portions of each bull ejaculate were processed and stained by two methods for comparison.

Control (no standardization, two step staining) - A control was established which did not include the step of standardizing collected ejaculates and in which the sperm was stained in two steps. Sperm in the control group was diluted to 160×10⁶ sperm per ml in a modified TALP buffer, as described in Table 1, at a pH of 7.4. Each sperm sample in the control group was then incubated with 16-17µL of Hoechst 33342 per ml (64-68 µM) of sample for 45 minutes at 34°C. After incubation, an equal volume of a second modified TALP was added reducing the concentration to 80×10⁶ sperm per mL. The second modified TALP includes the components described in Table 1 with the addition of 4% egg yolk, 50 µM red food dye No. 40 (20 g/L) and the pH was dropped to 5.5 with the addition of HCl.

Standardized III and One Step (standardized with 3% egg yolk extender, one step staining) - The remaining sperm was standardized by adjusting both the pH and sperm concentration prior to staining and sorting. After collection sperm was diluted 1:3 in an initial extender having a pH of 7.2 as well as a high capacity for buffering pH. The high capacity buffer was supplemented with 3% egg yolk. The sperm sample was then diluted to 160×10⁶ sperm per ml in a modified TALP buffer at a pH of 7.4. The modified TALP buffer was substantially identical to the buffer described in Table 1, except that it additionally included 1% egg yolk and yellow food dye No. 6 at a concentration of 25 µM. Each sperm sample in this group was then incubated with 14-15µL of Hoechst 33342 per ml (56-60 µM) for 45 minutes at 34°C. After incubation, sperm remained at a concentration of 160×10⁶ sperm per ml.

*Sorting and Results* - The control group was run through a Legacy MoFlo^{®} SX flow cytometer (Beckman Coulter, Maimi FL, US) with a Genesis digital upgrade (Cytonome/ST, Boston MA, USA) at a concentration of 80 ×10⁶ sperm per ml, while the Standardized III and One Step was sorted at a concentration of 160 ×10⁶ sperm per ml. Table 5 illustrates the percentage of cells in the dead gate of each ejaculate and the average. After sorting, percentages of sperm occurring in the dead gates (R2 seen in FIG. 3), were indicated for both samples. It can be seen the average over 17 bulls was 17% of the sperm was gated as dead in the control and only 10% of the sperm was gated as dead for the treated sperm, meaning the treatment provided a significant benefit to sperm health.

**TABLE 5**

| Bull | Dead Gate (%) | | |
|---|---|---|---|
| Ejaculate Number | Bull | CONTROL | ONE-STEP and STANDARDIZED III |
| 01 | Holstein Bull 1 | 16% | 12% |
| 02 | Holstein Bull 2 | 26% | 6% |
| 03 | Jersey Bull 1 | 15% | 7% |
| 04 | Holstein Bull 2 | 19% | 3% |
| 05 | Jersey Bull 1 | 13% | 6% |
| 06 | Holstein Bull 3 | 19% | 12% |
| 07 | Jersey Bull 2 | 25% | 14% |
| 08 | Holstein Bull 1 | 25% | 21% |
| 09 | Holstein Bull 2 | 20% | 20% |
| 10 | Jersey Bull 3 | 9% | 5% |
| 11 | Jersey Bull 2 | 19% | 17% |
| 12 | Holstein Bull 3 | 15% | 14% |
| 13 | Jersey Bull 1 | 10% | 7% |
| 14 | Holstein Bull 1 | 9% | 6% |
| 15 | Holstein Bull 1 | 9% | 8% |
| 16 | Holstein Bull 3 | 17% | 6% |
| 17 | Holstein Bull 3 | 16% | 5% |
| Average | | 17% | 10% |

### Example 4

*Collection and Sorting* - Sperm was collected from a Holstein bull and stained according to the Standardized III and One step protocol described in the Examples 1 and 3. The sample was placed on Legacy MoFlo^{®} SX flow cytometer (Beckman Coulter, Maimi FL, US) with a Genesis digital upgrade (Cytonome/ST, Boston MA, USA). During sorting, sheath fluid pressure was established at 40 psi and the drop drive frequency was set to 64.9KHz. The sample pressure was adjusted to target event rates of about 1500, 3500, 7500, 8500, 10,000 15000, 20000, 25000, and 30000.

*Results* - Measured sorting parameters from each target event rate were recorded in TABLE 6. The ejaculate in this example demonstrated about a 3%-5% dead gate which allowing for large portions of the sperm to be included in the live oriented gate; between 79.1% and 85.4%. The sorting logic utilized in this sort gated on a live oriented region of sperm (R1). R1 was established by an operator to retain a large portion of sperm. The X-sort gate was similarly established by an operator with a target of 90% purity. Data was periodically digitally logged for several samples at each event rate. Data was averaged at each event rate to provide averages for productivity (Sort Rate), sorting efficiency (Sort Rate/Event Rate), Valley to Peak ratio, abort rate, as well as the percentage of the population in the Dead gate (R2), the percentage of the population in the live oriented gate (R1), the percentage of the population of sperm in the X-Sort gate (R3), and the percentage of viable (live) sperm in the X-Sort Gate. Additionally, purities were determined off line for each sperm sorted at each event rate setting. Purities were determined by sonicating the tails off 1 million sperm and collected at each group of event rates and measurement in an offline purity analyzer. This measurement was performed twice for each group and averaged.

**TABLE 6**

| | Valley / Peak (%) | Event Rate (Hz) | Sort Rate (Hz) | Sort Rate / Event Rate (%) | Abort Rate (Hz) | Abort Rate / Sort Rate | Dead Gate (%) | Live-Oriented (%) | X-Sort Gate (%) | X-Sort / Viable (%) | X-Purity (%) |
|---|---|---|---|---|---|---|---|---|---|---|---|
| 1 | 67.4% | 1722 | 694 | 40.3% | 48 | 7.0% | 6.4% | 82.9% | 54.1% | 57.7% | 96.0% |
| 2 | 66.6% | 3697 | 1361 | 36.8% | 141 | 10.4% | 4.5% | 84.9% | 52.2% | 54.6% | 96.0% |
| 3 | 63.4% | 7377 | 2591 | 35.1% | 414 | 16.0% | 2.9% | 85.4% | 50.0% | 51.5% | 95.5% |
| 4 | 63.4% | 8515 | 3005 | 35.3% | 522 | 17.4% | 2.7% | 84.9% | 51.2% | 52.6% | 95.5% |
| 5 | 62.1% | 9891 | 3415 | 34.5% | 645 | 18.9% | 2.7% | 84.4% | 51.2% | 52.6% | 96.0% |
| 6 | 54.7% | 16686 | 4774 | 28.6% | 1306 | 27.4% | 2.8% | 82.8% | 47.1% | 48.5% | 93.0% |
| 7 | 51.0% | 19760 | 5080 | 25.7% | 1604 | 31.6% | 2.8% | 81.8% | 44.6% | 45.9% | 91.5% |
| 8 | 47.5% | 24839 | 5822 | 23.4% | 2175 | 37.4% | 2.8% | 80.2% | 43.5% | 44.8% | 90.0% |
| 9 | 43.9% | 29666 | 6332 | 21.3% | 2706 | 42.7% | 3.1% | 79.1% | 42.4% | 43.7% | 92.5% |

It can be seen that low event rates reduce the abort rates and improve sorting efficiency. In particular, the abort rate is 7% of the sort rate when the event rate is 1722.

Additionally the synergistic effect of reducing dead sperm is illustrated by virtue of the fact over 50% of the sperm sample was gated in the X-sort gate for event rates less than 10,000 events per second. The low percentage of dead sperm in combination with the high percentage of live oriented sperm allows gating an R3 region to be adjusted such that R3 encroaches the region of FIG. 4 where sperm have a greater probability of being Y-chromosomes bearing sperm than X-chromosome bearing sperm. Even when slightly encroaching this region, the purity checked post sort remained 96%, even though 54% of all sperm was included in the X-sort gate and 57% of all live sperm was included in the X-sort gate.

The synergistic combination of improved staining techniques in combination with sorting methods which focus on efficiency can be seen to provide reliable sperm sorting methods which may provide between 25% and about 40% yield on the total sperm population, and maintain purities greater than 90%.

One aspect of this disclosure projects more spatially efficient flow cytometers, which may allow more sorting heads in an available space. In such an arrangement, more flow cytometer sorting heads may be dedicated to a single sperm sample, and each one may be operated at an improved efficiency, thereby combining the benefits of efficient sorting methods with high productivity.

### Example 5

*Sperm handling* - A sperm sample was collected from five bulls including two Holstein bulls two Jersey bulls and one Simmental bull. At the time of collection, volume, concentration, motility, morphology and pH were checked, then antibiotics were added in accordance with industry practices. Each bull utilized in the example presented motility at or greater than 70%. The sperm sample was then standardized by placement in an extender with a pH buffering capacity and centrifuged for reconcentration between 1700-1800 million sperm per ml. 3ml of each bull were stained with TALP having Hochest 33342 and a yellow quenching food dye, and the concentrations after staining was 160 million sperm per ml, in accordance the one step staining described in previous examples.

Sperm from each bull was divided into four treatments. Two treatments were performed at 40 psi and two treatments were performed at 65 psi. At each of 40 and 65 psi, one treatment was established as a high productivity treatment and one treatment was established as a high efficiency treatment.

Treatment 1 - In the first treatment sorter sheath fluid pressure was set to 40 psi. An event rate of about 35,000 events per second was then established by adjusting the sample pressure. The drop drive frequency and other droplet generations signals were adjusted until a calibration side stream was established without spraying. A drop delay calibration was then performed to determine a charge delay.

Treatment 2 - In the second treatment the sorter was maintained at a pressure of 40 psi and the event rate was dropped to about 20,000 events per second with sample pressure adjustments. Gating was then readjusted on the flow cytometer.

Treatment 3 - In the third treatment the sheath fluid pressure was set to 65 psi and an event rate of about 35,000 events per second was established with the sample pressure. The drop drive frequency and other droplet generation signals were adjusted until a calibration side stream was established without spraying. A drop delay calibration was then performed to determine a charge delay.

Treatment 4 - In the fourth treatment sheath fluid pressure was maintained at 65 psi and an event rate of about 20,000 events per second was established by adjusting the sample pressure. Gating was then readjusted on the flow cytometer.

*Sperm Sorting and Freezing* - Sperm from each of the five bulls was sorted under each calibration described. During each sort, data was logged from the flow cytometer, and is seen in TABLE 7. A total of 10 million X-chromosome bearing sperm were collected in a catch tube having an A fraction of extender including about 20% egg yolk for each. Collected sperm was cooled for 90 minutes to about 5 C. B fraction of extender including 12% glycerol was added in two equal portions. After the B fraction was added, the sample was centrifuged and resuspended in an equal part A fraction and B fraction extender having about 20% egg yolk and about 6% glycerol. Multiple .25 ml straws were filled for each bull and each treatment and then frozen in liquid nitrogen.

**TABLE 7**

| | | Abort Rate | X Sort Rate | Oriented % | X Gate % | PVR |
|---|---|---|---|---|---|---|
| Treatment 1 | 40 psi | 2487 | 4490 | 60.38 | 36.77 | 43.82 |
| Treatment 2 | 40 psi | 1188 | 3460 | 63.03 | 38.79 | 52.42 |
| Treatment 3 | 65 psi | 2034 | 6019 | 62.65 | 39.27 | 48.48 |
| Treatment 4 | 65 psi | 982 | 4363 | 65.57 | 41.45 | 57.71 |

*Post Thaw* - Frozen straws were selected for each bull and treatment to undergo quality control testing. Motility was checked 0 hours and then again after three hours. Additionally, viability was determined by flow cytometer analysis of a portion of the thawed sperm that was then stained with Sybr Green and propidium iodide. The acrosome health of another portion of thawed sperm was analyzed by flow cytometry with PI/PNA staining. Additionally, sperm from each straw was sonicated and sperm nuclei were analyzed for purity. The results are seen in TABLE 8.

**TABLE8**

| | | 0hr Motility | 3hr Motility | Intact Acrosomes | Viable | Purity |
|---|---|---|---|---|---|---|
| Treatment 1 | 40 psi | 72 | 50 | 76 | 44 | 92 |
| Treatment 2 | 40 psi | 71 | 48 | 76 | 46 | 94 |
| Treatment 3 | 65 psi | 68 | 45 | 73 | 41 | 92 |
| Treatment 4 | 65 psi | 65 | 47 | 74 | 43 | 92 |

*Results* - The data logged in TABLE 7 illustrates several trends, a significant trend being that the slower event rates of treatments 2 and 4 slightly increased the percentage of sperm in the X gate and moderately improved the Peak to Valley ration (PVR) and the percentage of sperm in the oriented gate, as compared to treatments 1 and 3, respectively. Additionally, the increased pressure of treatment 3 and 4 independently decreased the abort rate and further improved the percentage of sperm in the X gate as compared to treatments 1 and 2, respectively.

### Example 6

*Sperm handling* - A sperm sample was collected from seven bulls including four Holstein bulls and three Jersey bulls. At the time of collection, volume, concentration, motility, morphology and pH were checked, then antibiotics were added. Each bull utilized in the example presented motility at or greater than 60%. The sperm sample was then standardized by placement in an extender with a pH buffering capacity and centrifuged for reconcentration between 1700-1800 million sperm per ml. Sperm was stained according to the One Step procedures outlines in Example 1, without the addition of egg yolk at the time of staining.

*Sorter Calibration* - A first data set was produced with a Legacy MoFlo^{®} SX flow cytometer having a Genesis digital upgrade available from Cytonome/ST (Boston, MA, USA) set to an operating sheath fluid pressure of 40 psi. The drop drive frequency was set to the highest frequency providing a good quality side stream within an existing recommended range. The drop delay was then determined with a test sort onto a microscope slide. The initial catch fluid level of a collection tube was positioned 4.5 inches below the deflection plates of the flow cytometer, or which is the standard position of a 50ml catch tube in a MoFlo^{®} flow cytometer.

A second dataset was produced with the same Legacy MoFlo^{®} SX flow cytometer operating with a sheath fluid pressure at 60 psi. The drop drive frequency was set to the highest frequency providing a good quality side stream within an existing recommended range. The drop delay was then determined with a test sort onto a microscope slide. The initial catch fluid level of a collection tube was positioned 4.5 inches below the deflection plates of the flow cytometer, or which is the standard position of a 50ml catch tube in a MoFlo^{®} flow cytometer.

A third data set was produced in the same manner as the second data set except that the collection tube was moved downwards 1.25 inches through a cut out in the work bench on which the flow cytometer was located. The initial level of the catch fluid was 5.75 inches below the deflection plates of the flow cytometer.

*Sperm Sorting and Freezing* - Sperm from each of the seven bulls was sorted under each calibration described. A total of 15 million X-chromosome bearing sperm were collected in a catch tube having an A fraction of extender including about 20% egg yolk for each.

Collected sperm was cooled for 90 minutes to about 5 C. B fraction of extender including 12% glycerol was added in two equal portions. After the B fraction was added, the sample was centrifuged and resuspended in an equal part A fraction and B fraction extender having about 20% egg yolk and about 6% glycerol. Multiple .25 ml straws were filled for each bull and each treatment and then frozen in liquid nitrogen.

*Post Thaw* - Frozen straws were later selected for each bull and treatment to undergo quality control testing. Straws were thawed and motility was checked at 0 hours and then again after three hours. Additionally, sperm viability was assessed by flow cytometry after staining with Sybr/PI. Five of the seven bulls were selected for IVF. Additionally sperm from each straw were sonicated and sperm nuclei were analyzed for purity.

*Results* - Averaged measured sorting parameters determined by data logging software were compiled for all sorts performed at 40 psi and for all sorts performed at 60 psi. Additionally, the average time to sort 15 million X chromosome bearing sperm at 40 psi was 48:17 and the average time to sort 15 million X chromosome bearing sperm at 60 psi was 34:23.

**TABLE 9**

| | Event Rate | Abort Rate | Sort X Rate | Oriented | Dead | X% | PVR |
|---|---|---|---|---|---|---|---|
| Avg.40 psi | 36,595 | 3034 | 5350 | 59.60 | 9.53 | 42.03 | 42.06 |
| Avg.60 psi | 40,830 | 2822 | 7175 | 60.32 | 9.68 | 43.82 | 46.39 |

The benefits of sorting at 60 psi over 40 psi can readily be seen in terms of productivity, as well as, efficiency in the averaged measured sorting parameters recorded in TABLE 9. With respect to productivity, an average of 7175 sorts per second allowed 15 million sperm to be sorted 13:54 faster. Further 60 psi, provided higher event rates, an improved sperm orientation, and an improved distinction between X chromosome bearing sperm and Y chromosome bearing sperm.

Each of the seven bulls were frozen, a straw for each bull was thawed and evaluated for motility, compromised sperm membranes (Sybr/PI) and purity. Five of the seven bulls, including three Holstein bulls and two Jersey bulls, were selected for IVF. The conversion of oocytes to embryos for each treatment is recorded in TABLE 11.

Additionally, a benefit was realized in changing the distance of the catch fluid, in particular for sorting at 60 psi. TABLE 10 illustrates the average post thaw motilities, viability and purity for each treatment over the five bulls selected for IVF trials.

**TABLE 10**

| | 0 Hr | 3 Hr | Viable | Purity |
|---|---|---|---|---|
| 40 psi - 4.5 | 59 | 38 | 31.66 | 92 |
| 60 psi - 4.5 | 56 | 35 | 30.59 | 94 |
| 60 psi - 5.75 | 65 | 39 | 32.60 | 93 |

Notably for the five bulls evaluated at 60 psi, the standard catch fluid location provided slightly lower post thaw motility and slightly lower viability as compared to sorting at 40 psi in the same location. However, moving the catch tube down an additional 1.25 inches (3.18 cm) provided a 10% improvement in 0 hour motility at 60 psi and an 11% improvement in 3 hour motility. For the five bulls utilized in IVF sperm sorted at 60 psi and collected at the second catch tube position demonstrated motility and viability which was slightly better than sorting at 40 psi.

**TABLE 11**

| | Oocytes | Embryos | % Oocytes converted to Embryos |
|---|---|---|---|
| 40 psi - 4.5 | 2004 | 205 | 10.23 |
| 60 psi - 4.5 | 2062 | 186 | 9.02 |
| 60 psi - 5.75 | 2081 | 194 | 9.32 |

### Example 7

A sperm sample was collected from five bulls. At the time of collection, volume, concentration, motility, morphology and pH were checked, then antibiotics were added. Each bull utilized in the example presented motility at or greater than 60%. The sperm sample was then divided into two groups.

Treatment I - The sperm sample in treatment I was stained in two steps, substantially in the same manner described above. In particular, treatment I was held as neat semen until it was stained in a first dilution to 160×10⁶ sperm per ml in a modified TALP buffer, as described in Table 1, at a pH of 7.4. Each sperm sample in the second group was then incubated with Hoechst 33342. After incubation, an equal volume of a second modified TALP was added reducing the concentration to 80×10⁶ sperm per mL. The second modified TALP includes the components described in Table 1 with the addition of red food dye No. 40, and 4% egg yolk and the pH was dropped to 5.5 with the addition of HCl.

Treatment II - The sperm sample in treatment II was then standardized with a 3 to 1 dilution in an initial extender, or a buffering holding extender. The buffering holding extender had a strong buffering capacity and a pH around 7.2. Additionally, antioxidants were added to the initial extender to reduce oxidative stresses in the sperm. The extended suspension was centrifuged and reconcentration to between 1600 ± 400 million sperm per ml. Sperm was stained in a single dilution to a concentration of 160×10⁶ in a modified TALP having BSA and being supplemented with an antioxidant regiment.

*Sperm Sorting* - A Genesis II flow cytometer available from Cytonome/ST (Boston, MA) was utilized to sort both stained samples. Sperm was sorted for the X-chromosome (female) in both treatments at event rates between 25,000 and about 40,000 events per second, depending on projected purity.

Sperm prepared according to treatment I, were sorted into a catch of TRIS citrate extender having 20% egg yolk (sometimes referred to as TRIS A).

Sperm from treatment II was sorted into a catch of TRIS citrate extender having 20% egg yolk with the addition of an antioxidant treatment.

*Post Sort*/*Freezing* - Sperm from treatment I was collected and cooled for 90 minutes to about 5 C. Tris B fraction of extender including 12% glycerol was added in two equal portions. After the B fraction was added, the sample was centrifuged and resuspended in an equal part A fraction and B fraction extender having about 20% egg yolk and about 6% glycerol. Multiple 0.25 ml straws were filled for each bull at a dosage of about 2.1 million sperm and each treatment and then frozen in liquid nitrogen.

Sperm from treatment II was collected and cooled for 90 minutes to about 5 C. Tris B fraction of extender including 12% glycerol and a combination of antioxidants to reduce oxidative stresses was added in two equal portions. After the B fraction was added, the sample was centrifuged and resuspended in an equal part A fraction and B fraction extender having about 20% egg yolk and about 6% glycerol and about 25 mg/µl antioxidants. Sperm from treatment II was divided into three groups. A first group was filled into 0.25 ml straws at a dosage of 2.1 million sperm per straw, a second group was filled into 0.25 ml straws at a dosage of 3 million sperm per straw, and a third group was filled into 0.25 ml straws at a dosage of 4 million sperm per straw and then frozen in liquid nitrogen.

Insemination was performed with each of the treatments and dosages in addition to a control of unsorted semen in a conventional dosage (15 million sperm per straw).

*Results* - For the first time the ill effects of flow cytometer sperm sorting were offset enough so that a dose response effect with sex-sorted sperm could be seen. Additionally, for the first time, a dosage of sex sorted sperm was shown to perform as well as conventional dosages of unsexed sperm. In Table 12, a 56 day non-return rate (NRR) was compared for each treatment and dosage. Additionally, relative fertility was compared as a percentage to the conventional insemination.

**TABLE 12**

| Treatment | Number of Inseminations | 56 day NRR (%) | Relative fertility |
|---|---|---|---|
| Treatment I | 1953 | 55.9^{A} | 84% |
| Treatment II - 2.1 mil | 1999 | 59.9^{B} | 90% |
| Treatment II - 3 mil | 2013 | 60.0^{B} | 90% |
| Treatment II - 4 mil | 1890 | 66.7^{C} | 100% |
| Conventional - 15 mil | 62,398 | 66.5^{C} | ---- |

Data from cows and heifers. NRR results with different subscripts are significantly different P<0.05

Having now established a sperm sorting methodology which provides a sex sperm capable of demonstrating a dose response, it is expected further dosage increases may begin to surpass conventional semen fertility. While sex sorting is an injurious procedure for sperm, the sorting step actively removes dead or membrane compromised sperm cells from the sorted sperm sample. It may be that in the improved sorting method those injurious steps of sperm sorting process irreparably harm those sperm cells that were less likely fertilize an egg thereby removing the most subfertile sperm from the insemination sample. As such, once the injuries of the overall process have been reduced sufficiently to provide a dose response, further increases in sperm dosages may begin to surpass conventional unsorted semen by virtue of removing the most subfertile sperm from the sorted population.

## Claims

1. A method of producing sex sorted bovine sperm suitable for insemination having an improved dose response comprising:
a) extending a bovine sperm sample with a buffering holding media at a sperm sample to buffering holding media ratio between about 1:1 and 1: 10;
b) adjusting the concentration of the extended sperm sample to a target concentration range and adjusting the pH of the sperm sample towards a predetermined pH by centrifuging the extended sperm sample and removing supernatant to a concentration between about 2400 million sperm per ml and about 500 million sperm per ml, the predetermined pH being a target pH range from 6.8 to 7.4;
c) diluting the reconcentrated sperm with a staining media to a concentration between 80 million sperm per ml and 320 million sperm per ml;
d) staining the reconcentrated sperm sample with a DNA selective dye;
e) maintaining the pH of the sperm sample through steps a) to d) at the target pH range;
f) sex sorting the stained sperm sample with a flow cytometer into a catch fluid; and
g) supplementing each of the buffering holding media, the DNA selective dye and the catch fluid with at least one antioxidant at a concentration between 0.01 mg per ml and 0.5 mg per ml;
h) producing an insemination dosage having between 3 million and 4 million sperm and having at least 90% of the fertility of a conventional dose of 15 million sperm.

2. The method of claim 1, wherein the steps of extending a sperm sample with a buffering holding media and adjusting the concentration of the extended sperm sample to a target concentration range further comprise: a) extending the sperm sample in the buffering holding media at a target pH between 7.0 and 7.4; and b) concentrating the extended sperm sample to a target concentration between about 800 × 10⁶ sperm per ml and about 2100 × 10⁶ sperm per ml.

## Patentansprüche

1. Verfahren zur Erzeugung von geschlechtssortiertem Rindersperma, geeignet zur Besamung, das eine verbesserte Dosisreaktion aufweist, das Folgendes umfasst:
a) Strecken einer Rinderspermaprobe mit einem buffernden Haltemedium in einem Verhältnis von Spermaprobe zu pufferndem Haltemedium von zwischen etwa 1 : 1 und 1 : 10;
b) Einstellen der Konzentration der gestreckten Spermaprobe auf einen Zielkonzentrationsbereich und Einstellen des pH der Spermaprobe auf einen vorgegebenen pH durch Zentrifugieren der gestreckten Spermaprobe und Entfernen des Überstands, auf eine Konzentration zwischen etwa 2400 Millionen Spermien pro ml und etwa 500 Millionen Spermien pro ml, wobei der vorgegebene pH ein Ziel-pH-Bereich von 6,8 bis 7,4 ist;
c) Verdünnen des aufkonzentrierten Spermas mit einem Färbemedium auf eine Konzentration zwischen 80 Millionen Spermien pro ml und 320 Millionen Spermien pro ml;
d) Einfärben der aufkonzentrierten Spermaprobe mit einem DNA-selektiven Farbstoff;
e) Aufrechterhalten des pH der Spermaprobe während der Schritte a) bis d) in dem Ziel-pH-Bereich;
f) Geschlechtssortierung der eingefärbten Spermaprobe mit einem Durchflusszytometer in eine Auffangflüssigkeit; und
g) Ergänzen des puffernden Haltemediums, des DNA-selektiven Farbstoffs und der Auffangflüssigkeit jeweils mit mindestens einem Antioxidationsmittel bei einer Konzentration zwischen 0,01 mg pro ml und 0,5 mg pro ml;
h) Erzeugen einer Besamungsdosierung, die zwischen 3 Millionen und 4 Millionen Spermien aufweist und mindestens 90 % der Fertilität einer konventionellen Dosis von 15 Millionen Spermien aufweist.

2. Verfahren nach Anspruch 1, wobei die Schritte des Streckens einer Spermaprobe mit einem puffernden Haltemedium und des Einstellens der Konzentration der gestreckten Spermaprobe auf einen Zielkonzentrationsbereich weiter Folgendes umfassen: a) Strecken der Spermaprobe in dem puffernden Haltemedium bei einem Ziel-pH-Bereich zwischen 7,0 und 7,4; und b) Konzentrieren der gestreckten Spermaprobe auf eine Zielkonzentration zwischen etwa 800 × 10⁶ Spermien pro ml und etwa 2100 × 10⁶ Spermien pro ml.

## Revendications

1. Méthode de production de spermatozoïdes bovins triés par sexe, convenables à des fins d'insémination et présentant une dose-réponse améliorée, comprenant :
a) l'extension d'un échantillon de sperme bovin par un milieu de rétention tampon selon un rapport d'échantillon de sperme/milieu de rétention tampon compris entre environ 1:1 et 1:10 ;
b) l'ajustement de la concentration de l'échantillon de sperme étendu jusqu'à une plage de concentration cible et l'ajustement du pH de l'échantillon de sperme jusqu'à un pH prédéterminé par la centrifugation de l'échantillon de sperme étendu et l'élimination du surnageant jusqu'à une concentration comprise entre environ 2400 millions de spermatozoïdes par ml et environ 500 millions de spermatozoïdes par ml, le pH prédéterminé étant une plage de pH cible allant de 6,8 à 7,4 ;
c) la dilution du sperme reconcentré par un milieu de coloration jusqu'à une concentration comprise entre 80 millions de spermatozoïdes par ml et 320 millions de spermatozoïdes par ml ;
d) la coloration de l'échantillon de sperme reconcentré par un colorant sélectif de l'ADN ;
e) le maintien du pH de l'échantillon de sperme au cours des étapes a) à d) au niveau de la plage de pH cible ;
f) le tri, en fonction du sexe, de l'échantillon de sperme coloré à l'aide d'un cytomètre en flux dans un liquide de capture ; et
g) la complémentation de chacun parmi le milieu de rétention tampon, le colorant sélectif de l'ADN et le liquide de capture, par au moins un antioxydant selon une concentration comprise entre 0,01 mg par ml et 0,5 mg par ml ;
h) la production d'un dosage d'insémination contenant entre 3 millions et 4 millions de spermatozoïdes et présentant au moins 90% de la fertilité d'une dose conventionnelle de 15 millions de spermatozoïdes.

2. Méthode selon la revendication 1, dans laquelle les étapes d'extension d'un échantillon de sperme par un milieu de rétention tampon et d'ajustement de la concentration de l'échantillon de sperme étendu jusqu'à une plage de concentration cible comprennent en outre : a) l'extension de l'échantillon de sperme dans le milieu de rétention tampon jusqu'à un pH cible compris entre 7,0 et 7,4 ; et b) la concentration de l'échantillon de sperme étendu jusqu'à une concentration cible comprise entre environ 800 × 10⁶ spermatozoïdes par ml et environ 2100 × 10⁶ spermatozoïdes par ml.
